Europäisches Patentamt

European Patent Office

Office européen des brevets

(19)

(11)     EP 1 131 459 B1

(12)                        EUROPEAN PATENT SPECIFICATION

(45) Date of publication and mention
     of the grant of the patent:
     **30.07.2003  Bulletin 2003/31**

(51) Int Cl.7: **C12P 3/00**

(86) International application number:
     **PCT/NL99/00705**

(21) Application number: **99956353.9**

(22) Date of filing: **16.11.1999**

(87) International publication number:
     **WO 00/029605 (25.05.2000 Gazette 2000/21)**

(54) **PROCESS FOR THE PRODUCTION OF HYDROGEN SULPHIDE FROM ELEMENTAL SULPHUR AND USE THEREOF IN HEAVY METAL RECOVERY**

VERFAHREN ZUR ERZEUGUNG VON SCHWEFELWASSERSTOFF AUS ELEMENTAREM SCHWEFEL UNS IHRE VERWENDUNG ZUR RÜCKGEWINNUNG VON SCHWERMETALLEN

PROCEDE DE PRODUCTION DE SULFURE D'HYDROGENE A PARTIR DE SOUFRE ELEMENTAIRE, ET UTILISATION DE CE SULFURE DANS LA RECUPERATION DE METAUX LOURDS

(84) Designated Contracting States:
     **AT BE CH CY DE DK ES FI FR GB GR IE IT LI LU MC NL PT SE**

(30) Priority:  **16.11.1998  EP 98203853**
     **29.12.1998  EP 98204462**

(43) Date of publication of application:
     **12.09.2001  Bulletin 2001/37**

(73) Proprietor: **Paques Bio Systems B.V.**
     **8560 AB Balk (NL)**

(72) Inventors:
     • **BUISMAN, Cees, Jan, Nico**
       **NL-8571 RH Harich (NL)**

• **DIJKMAN, Henk**
  **NL-8651 AA Ijlst (NL)**

(74) Representative: **van Westenbrugge, Andries et al**
     **Nederlandsch Octrooibureau**
     **P.O. Box 29720**
     **2502 LS The Hague (NL)**

(56) References cited:
     **EP-A- 0 778 350**      **EP-A- 0 819 756**
     **WO-A-92/17410**       **DE-A- 3 321 515**
     **DE-A- 19 529 098**     **US-A- 5 670 123**

## Description

### Technical field

The present invention is concerned with the production of hydrogen sulphide from elemental sulphur under mild conditions and with the use of the hydrogen sulphide in metal recovery.

### Background

Hydrogen sulphide is an expensive chemical used in the metallurgical and mining industry, but also in the electronic industry. It is used for example in nickel, zinc and copper mining and metallurgical operations for selective recovery and removal of metals from leach water streams, acid plant blow down streams, refinery electrolyte bleeds and precious metal plant bleeds. The required sulphide is normally:

(i) produced on site by catalytic reduction of elemental sulphur at elevated pressure and temperatures (above 150 °C) or transported to the site as liquefied hydrogen sulphide ($H_2S$) afterwards; such processes of chemical hydrogen sulphide production are known, e.g. from U.S. patents 4,094,961, 4,146,580 and 4,332,774;
(ii) transported to the site as a sodium sulphide solution (NaHS); or
(iii) produced on site by the biological reduction of sulphate from diluted waste water streams (see e.g. WO 97/29055, US 5,587,079).

These methods have disadvantages in that they are relatively expensive, require catalysts, introduce high salt, alkali or acid loads, and require more extensive safety precautions. WO 92/17410 discloses a process for removing sulphur compounds from water by anaerobic treatment with sulphur-reducing bacteria at high temperatures.

### Description of the invention

An improved process for the production of hydrogen sulphide has been found now. The process of the invention uses elemental sulphur, which is biologically reduced to hydrogen sulphide. The process has the following advantages compared to the above mentioned methods:

(a) Biological reduction of elemental sulphur can be carried out at mild temperature and pressure. Compared to catalytic reduction on site, the biological reduction is much safer and cheaper. Another advantage of the new process is that the production of sulphide is possible on a demand basis. The biological production of sulphide can be turned off and on very easily.
(b) Compared to adding a sodium sulphide solution (NaHS) in processes where sulphide is required, the biologically produced sulphide is less expensive and it has the advantage that no sodium enters the processes involved. Very often a low pH is required in the metallurgical operations, implying that more acid will have to be added when also sodium is added to the process. Furthermore the sodium will end up in a waste water stream as sodium sulphate which will has to be discharged or treated. Traditional lime treatment in this case however will not remove the sulphates as efficiently as before due to the presence of the sodium.
(c) Compared to the biological production of sulphide from a diluted sulphate containing waste water stream, the process of the invention has several advantages. It is much less expensive, due to the fact that only 25 % of the amount of electron donor (hydrogen gas or organic compounds) is required for the reduction of elemental sulphur compared to the reduction of sulphate. Another advantage is that the pH in the bioreactor can be kept low, enabling a more efficient removal of the produced hydrogen sulphide from the liquid. Also, in the process of the invention water can be recycled to minimise the sulphide-containing liquid effluent to zero. In case of producing sulphide from sulphate present in a diluted waste water stream normally a post-treatment is required to oxidise the dissolved sulphide present in the effluent of the anaerobic reactor. Another advantage is that due to the fact that no liquid bleed is required; thus, bacteria can be retained in the reactor without a biomass retention system being necessary. This increases the active biomass concentration significantly and results in higher sulphide production rates.

The process of the invention can be carried out using a bioreactor which is fed with a concentrated elemental sulphur stream and an electron donor for the biological reduction of the sulphur to produce hydrogen sulphide. As electron donor gaseous components like hydrogen and carbon monoxide can be used but also organic compounds such as ethanol, methanol, acetic acid or other fatty acids.

**Biological characteristics**

| The bacteria: | The biological reduction of sulphur is accomplished by a mixed culture of unidentified sulphur reducing bacteria such as species from the genera: *Desulforomonas sp.* (mesophilic), *Desulfotomaculum* KT7 (thermophilic), the species *Desulforolobus ambivalens, Acidianus infernus, Acidianus brierley, Stygiolobus azoricus* (mesophilic), *Thermoproteus neutrophilus, Thermoproteus tenax, Thermodiscus maritimus* (thermophilic), *Pyrobaculum islandicum, Pyrodictium occultum, Pyrodictium brockii* (hyperthermophilic), and other species of the genera *Desulfovibrio, Desulfotomaculum, Desulfomonas, Desulfobulbus, Desulfobacter, Desulfococcus, Desulfonema, Desulfosarcina, Desulfobacterium* and *Desulforomas* (mesophilic), and species of sulphur-reducing methanogenic bacteria such as form the genera *Methanococcus* and *Methanobacterium.* |
| The electron donor: | Hydrogen gas, carbon monoxide, alcohols (e.g. ethanol, methanol), fatty acids (e.g. acetic acid) or other readily degradable organic compounds. |

The biological conversions:

| Hydrogen gas as electron donor : | $H_2 + S° \rightarrow H_2S$ |
| Organic compounds as electron donor e.g. ethanol: | $C_2H_5OH + 6\,S° + 3\,H_2O \rightarrow 6\,H_2S + 2\,CO_2$ |
| Temperature: | The process can be operated under mesophilic conditions (15-40°C) or under thermophilic conditions (40-90°C). The preferred temperature ranges are 25-75°C. Mesophilic temperatures for use with hydrogen are a particular aspect of the invention. |
| The pH: | Operating between pH 5 and 9, preferably between 6 and 8.5, most preferably between 6 and 8. |

Metals such as copper, zinc, nickel, cobalt, tin, lead, cadmium, bismuth, mercury, silver, iron, manganese, chromium, vanadium and titanium, can be recovered by contacting the hydrogen sulphide produced according to the invention with a liquid containing the metals and precipitating the metals as their sulphides. Such metals can also be recovered selectively using e.g. varying pH's as described in WO 97/29055.

After dewatering, the concentrated metal sulphide sludge can be processed using conventional metallurgical processes to recover the pure metal. For example, copper and zinc sulphides can be converted to elemental copper and zinc in roosting and melting processes combined with electrowinning. Especially favourable is the ECUPREX®-EW process for recovery of copper and lead, as this process produces elemental sulphur as a side product, and this side product can be used again for the production of the required hydrogen sulphide. This process is described in EP-A-411687. Copper and lead can thus be recovered from waste water or process streams by precipitation with hydrogen sulphide as insoluble copper sulphide or lead sulphide according to:

$$1. \quad CuSO_4 + H_2S \rightarrow CuS + H_2SO_4$$

According to the ECUPREX®-EW process these metal sulphides are settled and dewatered and are then contacted with a fluoroboric leaching solution in which the metal dissolves and the sulphide is oxidised to elemental sulphur according to :

$$2. \quad CuS + 2\,Fe(BF_4)_3 \rightarrow Cu(BF_4)_2 + 2Fe(BF_4)_2 + S°$$

After separation of the elemental sulphur, electrolysis is used to produce pure copper at the cathode and to reoxidise the iron at the anode according to :

$$3. \quad Cu(BF_4)_2 + 2\ e^- \rightarrow Cu + 2\ BF_4^-$$

$$2\ Fe(BF_4)_2 + 2\ BF_4^- \rightarrow 2\ Fe(BF_4)_3 + 2\ e^-$$

After dewatering the sulphur slurry obtained in the leaching step (step 2) can be used for biological hydrogen sulphide production and the produced hydrogen sulphide is used again for the metal precipitation in step 1. In this way a perfect sulphur cycle has been created in which no sulphur compounds have to be purchased for the production of the hydrogen sulphide required to precipitate the metals in step 1.

## Engineering characteristics

*Process design*

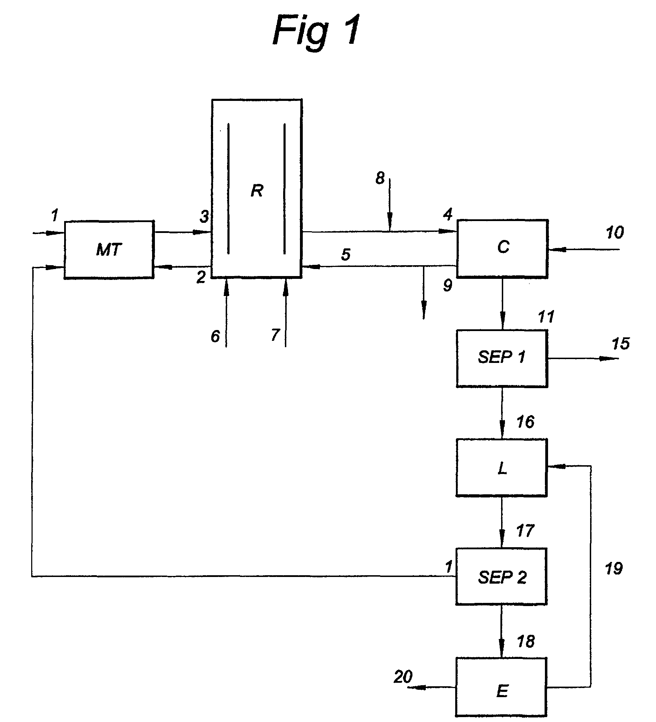

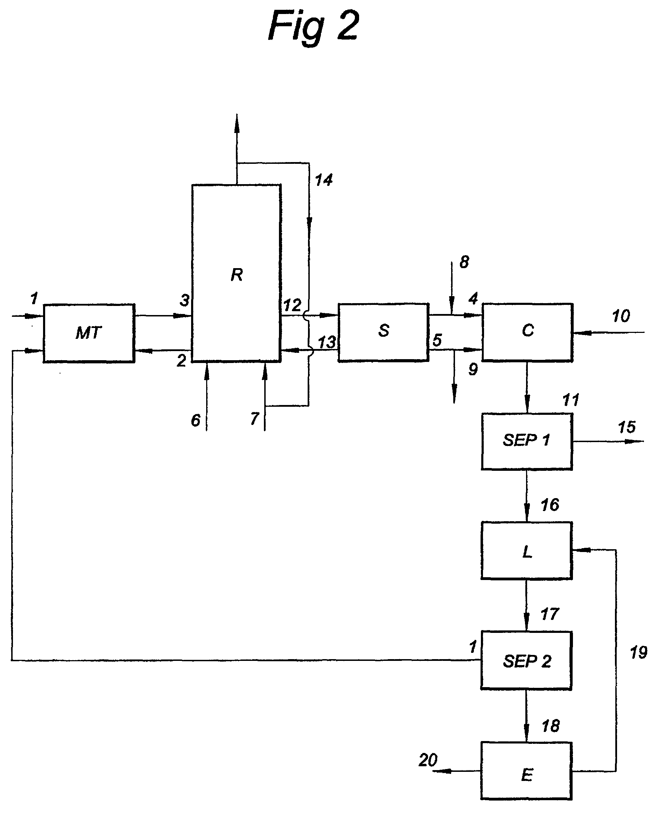

Figure 1 shows a possible set-up for the process of the invention. Elemental sulphur (1) is preferably added in the form of ground particles which are either added directly to the reactor or preferably slurried up in the mixing tank (MT) using part of the reactor liquid (3) for this purpose prior to addition (2) to the bioreactor (R). In the anaerobic bioreactor an electron donor (organic compound (6) or hydrogen/carbon monoxide (7)) is added and the elemental sulphur is reduced to produce hydrogen sulphide under ambient conditions. The bioreactor is well mixed in order to suspend the biomass and sulphur particles in the reactor and to create an effective contact between the two. Also the mixing prevents gradients of dissolved sulphide concentrations and pH through the reactor. Mixing can be achieved by different means, although it is preferred to mix the reactor using a gas recycle stream (4,5). Preferably a gas-lift loop type of reactor is used in this case to optimise the mixing characteristics of the reactor. A gas recycle is preferred for mixing because it provides an easy way to control the pH in the reactor and remove the produced hydrogen sulphide from the reactor by means of contacting the recycle gas with a process stream in which the hydrogen sulphide is required. Another possible flow scheme would be to lead part of the liquid effluent (12) of the anaerobic bioreactor to a separate sulphide stripping column (S) with recycle (13) and remove the sulphide from this stream by contacting it either with a process gas stream (14) or a gas recycle stream (4,5) over the contactor (C). This is shown in figure 2. A disadvantage of this flow scheme however is that the hydrogen sulphide is not removed from the liquid in the reactor itself. Applying a same pH in the bioreactor this will mean that the pH in the stripper will rise due to the removal of hydrogen sulphide resulting in a higher gas recycle flow required over the contactor unit to transport the same amount of hydrogen sulphide.

The contactor (C) is a device in which the hydrogen sulphide containing recycle gas is contacted with a process stream (10) to transfer the hydrogen sulphide from the recycle gas to the process stream. The design of the contactor and the process streams involved may be different for different applications. For example the contactor could be an open spray tower in which a metal containing process stream is contacted with the recycle gas. Metals will precipitate as metal sulphides and can be separated from the process stream downstream the contactor.

The process stream (10) may advantageously be a metal-containing stream (10) to which the hydrogen sulphide from the recycle gas is transferred. The metals will precipitate and the solid can be removed from the liquid downstream (11) the contactor in a solids separator (SEP 1). The design of the gas-liquid contactor is mainly dependent on gas flow rate, the liquid flow rate, the hydrogen sulphide concentration in the gas and the metal concentration in the liquid. An open spray tower or a packed column could e.g. be used for this purpose. After settling of the metal sulphides, the treated water (15) is discharged and the metal sulphides (16) are dewatered and can then be processed using conventional metallurgical processes to recover the pure metal. In case of the ECUPREX-EW process, the dewatered copper or lead sulphide sludge is contacted with the fluoroboric leaching solution (19) in the leaching reactor (L). In the second liquid-solids separator (SEP 2) the elemental sulphur (1) is separated from stream (17) and returned to the bioreactor (R). The dissolved metal containing solution (18) is led to the electrolysis unit (E) in which the pure metal is produced (20) and the fluoroboric leaching solution (19) is regenerated.

Instead of a metal-containing liquid, the process stream (10) fed to the contactor (C) may also be a liquid which absorbs the hydrogen sulphide in order to transport it to a regeneration column to concentrate the hydrogen sulphide to a higher percentage gas stream (> 90%). This hydrogen sulphide gas stream can be used for the different industrial purposes of hydrogen sulphide. Furthermore, the contactor (C) may also be a membrane unit in which the hydrogen sulphide is selectively removed and concentrated to produce also a more highly concentrated gas stream to be used for different purposes in industry. It is also possible to strip the hydrogen sulphide directly from the bioreactor (R) or from stripper (S) using a process gas stream instead of using a gas recycle to transport the hydrogen sulphide to a liquid stream.

*Process control*

In order to achieve high hydrogen sulphide production rates the process has to be controlled carefully. An important factor is the control of the pH in the water system (the water system comprises the reactor and optional stripper and connecting lines). The biological reduction of elemental sulphur using hydrogen gas, carbon monoxide or organic compounds as an electron donor results in the production of the acids hydrogen sulphide and carbon dioxide in the bioreactor. In principle this would lower the pH in the reactor to low levels which may inhibit the biological reactions. As no liquid bleed stream from the water system is desired, increasing the pH in the reactor by continuous addition of alkaline components such as sodium hydroxide is undesirable as the sodium concentration would build up to unacceptable levels inducing a liquid bleed stream at some level. The pH in the system is controlled by removing the acids from the liquid by stripping either directly from the reactor (figure 1) or from the effluent of the reactor (figure 2) in combination with the removal of the acid components from the recycle gas. The absence of a liquid bleed corresponds to a long hydraulic retention time of at least 1 day, preferably at least 5 days, up to 1 month or more, depending on the amount of water introduced with the elemental sulphur.

The process is a continuously operating system and all hydrogen sulphide produced is eventually removed from the liquid. However, the pH and the dissolved hydrogen sulphide concentration in the reactor can be selected freely. It can be maintained at a specific level by adjusting the electron donor feed to the removal of hydrogen sulphide in the contactor. The hydrogen sulphide concentration in the gas is kept high to be able to reduce the gas recycle flow for transporting the hydrogen sulphide and enhance mass transfer in the contactor. The hydrogen sulphide concentration in the gas is at least 1 vol.%, preferably at least 3 vol.%, more preferably at least 10 vol.%. As there is little or no liquid bleed from the bioreactor system, active biomass concentrations can be increased easily without installing a biomass retention system. This allows for higher tolerable dissolved hydrogen sulphide concentrations in the gas. Dissolved sulphide concentrations in the bioreactor are preferably at least 300 mg/l, especially at least 600 mg/l up to 3000 mg/l. Dissolved hydrogen sulphide concentrations above 3000 mg/l (above 20% in the gas) can be reached without loss of sulphide production capacity in the bioreactor. The biological production of hydrogen sulphide can be turned off instantaneously by interrupting the removal of hydrogen sulphide from the contactor. The hydrogen sulphide concentration will increase then and eventually inhibit the production of hydrogen sulphide. The process is reversible. Thus, after starting the removal of hydrogen sulphide again, the concentration in the bioreactor will decrease and hydrogen sulphide production will resume immediately. Another way of stopping and starting the production instantaneously is by stopping and continuing the electron donor supply.

Hydrogen sulphide is removed as an acid in the contactor (C) and the produced carbon dioxide is removed by purging part of the gas (9) from the gas recycle system. The amount of carbon dioxide purged can be controlled by controlling the amount of an inert gas e.g. nitrogen gas (8) added to the recycle gas for this reason. The surplus gas resulting from the carbon dioxide purge is separated from the hydrogen sulphide and then removed.

**Example 1:**

In a 5 litre gas lift loop reactor 1.25 g/h of ground elemental sulphur was added by means of pumping from a tank in which the ground sulphur was mixed with liquid from the bioreactor.

Hydrogen gas was added as electron donor for the biological reduction. No make-up water was used and no liquid bleed existed. The reactor was operated at 35°C and the pH in de reactor was maintained at 7.5 by stripping the hydrogen sulphide from the liquid with the recycle gas. The recycle gas was contacted with leach water (7 litre/h) containing copper removing the acid hydrogen sulphide from the recycle gas and recovering the copper as copper sulphide from the leach water. Dissolved hydrogen sulphide concentrations up to 2000 mg/l were found. The hydrogen sulphide concentration in the gas reached up to 15%.

**Example 2:**

In a 8 litre laboratory reactor 80 grams of ground elemental sulphur was added daily and ethanol was continuously added as electron donor for the reduction. The reactor was operated at 30°C and the pH was controlled at pH 7 applying a gas recycle to strip out the hydrogen sulphide and carbon dioxide. The hydrogen sulphide was removed from the recycle gas by contacting the gas with a copper sulphate containing solution in a bubble column. Carbon dioxide was removed by adding small amount of nitrogen gas to the recycle gas creating a gas purge stream containing nitrogen, carbon dioxide and small amounts of hydrogen sulphide. Dissolved hydrogen sulphide concentrations up to 1500 mg/l were found. The hydrogen sulphide concentration in the gas reached up to 20%.

**Claims**

1. A process for the production of hydrogen sulphide for industrial use by reduction of a sulphur source in a liquid medium in a bioreactor, using sulphur-reducing bacteria as a catalyst and hydrogen gas, carbon monoxide or organic compounds as an electron donor, *characterised by* using elemental sulphur as the sulphur source, using a hydraulic retention time of the liquid medium of at least 1 day and a pH between 5 and 9 and stripping the hydrogen sulphide from the liquid medium to produce a gas containing at least 1 vol.% of hydrogen sulphide.

2. A process according to Claim 1, in which the hydrogen sulphide is stripped from the bioreactor, at such a rate that a pH between 6 and 8.5 is maintained in the bioreactor.

3. A process according to Claim 1 or 2, in which a sulphide gas containing at least 3 vol.%, preferably at least 10 vol.% of hydrogen sulphide is produced.

4. A process according to any one of Claims 1-3, in which the hydraulic retention time is at least 5 days.

5. A process according to any one of the preceding Claims, in which carbon dioxide is also stripped from the liquid medium by means of addition of an inert gas and subsequently separated from hydrogen sulphide.

6. A process according to any one of the preceding Claims, in which the hydrogen sulphide in the produced gas is concentrated using a membrane unit or an absorption/regeneration unit.

7. A process according to any one of the preceding Claims, in which a sulphide concentration of at least 300 mg/l, especially at least 600 mg/l, up to 3000 mg/l is maintained in the bioreactor.

8. A process according to any one of the preceding Claims, in which hydrogen is used as an electron donor.

9. A process according to Claim 8, in which a temperature of 15-40°C is maintained in the bioreactor.

10. A process according to any one of the preceding Claims, in which the sulphide gas is subsequently contacted with a heavy metal containing stream and metals are precipitated as metal sulphides.

11. A process according to Claim 10, in which the metal sulphides are subsequently treated to produce metals and elemental sulphur, and the elemental sulphur is recycled to the reduction step in the bioreactor.

12. A process according to Claim 10 or 11, in which the heavy metal comprises copper and/or lead.


**Patentansprüche**

1. Verfahren zur Herstellung von Schwefelwasserstoff zur industriellen Verwendung durch Reduktion einer Schwefelquelle in einem flüssigen Medium in einem Bioreaktor durch Verwenden schwefelreduzierender Bakterien als Katalysator und Wasserstoffgas, Kohlenmonoxid oder organischer Verbindungen als Elektronendonor, **gekennzeichnet durch** das Verwenden elementaren Schwefels als Schwefelquelle, das Anwenden einer Flüssigkeitsrückhaltezeit des flüssigen Mediums von mindestens 1 Tag und eines pH zwischen 5 und 9 und Abziehen des Schwefelwasserstoffs aus dem flüssigen Medium unter Erzeugen eines mindestens 1. Vol.-% Schwefelwasserstoff enthaltenden Gases.

2. Verfahren gemäß Anspruch 1, wobei der Schwefelwasserstoff aus dem Bioreaktor mit einer solchen Geschwindigkeit abgezogen wird, daß in dem Bioreaktor ein pH zwischen 6 und 8,5 aufrecht erhalten wird.

3. Verfahren gemäß Anspruch 1 oder 2, wobei ein mindestens 3 Vol.-%, vorzugsweise mindestens 10 Vol.-% Schwefelwasserstoff enthaltendes Sulfidgas erzeugt wird.

4. Verfahren gemäß einem der Ansprüche 1-3, wobei die Flüssigkeitsrückhaltezeit mindestens 5 Tage beträgt.

5. Verfahren gemäß einem der vorangehenden Ansprüche, wobei auch Kohlendioxid aus dem flüssigen Medium durch Zusatz eines Inertgases abgezogen und nachfolgend von dem Schwefelwasserstoff abgetrennt wird.

**6.** Verfahren gemäß einem der vorangehenden Ansprüche, wobei der Schwefelwasserstoff in dem erzeugten Gas mittels einer Membraneinheit oder einer Absorptions-/Regenerierungseinheit konzentriert wird.

**7.** Verfahren gemäß einem der vorangehenden Ansprüche, wobei in dem Bioreaktor eine Sulfidkonzentration von mindestens 300 mg/l, insbesondere mindestens 600 mg/l und bis zu 3000 mg/l aufrecht erhalten wird.

**8.** Verfahren gemäß einem der vorangehenden Ansprüche, wobei Wasserstoff als Elektronendonor verwendet wird.

**9.** Verfahren gemäß Anspruch 8, wobei in dem Bioreaktor eine Temperatur von 15-40°C aufrecht erhalten wird.

**10.** Verfahren gemäß einem der vorangehenden Ansprüche, wobei das Sulfidgas nachfolgend mit einem schwermetallhaltigen Strom in Kontakt gebracht wird und die Metalle als Metallsulfide gefällt werden.

**11.** Verfahren gemäß Anspruch 10, wobei die Metallsulfide nachfolgend zum Herstellen von Metallen und elementarem Schwefel behandelt werden und der elementare Schwefel in den Reduktionsschritt in dem Bioreaktor zurückgeführt wird.

**12.** Verfahren gemäß Anspruch 10 oder 11, wobei das Schwermetall Kupfer und/oder Blei umfaßt.

**Revendications**

**1.** Procédé de production d'hydrogène sulfuré pour usage industriel par réduction d'une source de soufre en milieu liquide dans un bioréacteur, en utilisant des bactéries réductrices du soufre comme catalyseur et de l'hydrogène gazeux, du monoxyde de carbone ou des composés organiques comme donneur d'électrons, *caractérisé par* l'utilisation de soufre élémentaire comme source de soufre, en utilisant un temps de rétention hydraulique du milieu liquide d'au moins 1 jour et un pH entre 5 et 9 et en lavant l'hydrogène sulfuré du milieu liquide pour produire un gaz contenant au moins 1 vol. % d'hydrogène sulfuré.

**2.** Procédé selon la revendication 1, dans lequel l'hydrogène sulfuré est lavé du bioréacteur, à une vitesse telle qu'un pH entre 6 et 8,5 est maintenu dans le bioréacteur.

**3.** Procédé selon la revendication 1 ou 2, dans lequel un gaz sulfuré contenant au moins 3 vol. %, de préférence au moins 10 vol. % d'hydrogène sulfuré est produit.

**4.** Procédé selon l'une quelconque des revendications 1-3, dans lequel le temps de rétention hydraulique est d'au moins 5 jours.

**5.** Procédé selon l'une quelconque des revendications précédentes, dans lequel le dioxyde de carbone est également lavé du milieu liquide au moyen de l'addition d'un gaz inerte et est ensuite séparé de l'hydrogène sulfuré.

**6.** Procédé selon l'une quelconque des revendications précédentes, dans lequel l'hydrogène sulfuré dans le gaz produit est concentré en utilisant une unité à membrane ou une unité d'absorption/régénération.

**7.** Procédé selon l'une quelconque des revendications précédentes, dans lequel une concentration en sulfure d'au moins 300 mg/l, en particulier d'au moins 600 mg/l, jusqu'à 3000 mg/l est maintenue dans le bioréacteur.

**8.** Procédé selon l'une quelconque des revendications précédentes, dans lequel l'hydrogène est utilisé comme donneur d'électrons.

**9.** Procédé selon la revendication 8, dans lequel une température de 15-40°C est maintenue dans le bioréacteur.

**10.** Procédé selon l'une quelconque des revendications précédentes, dans lequel le gaz sulfuré est ensuite mis en contact avec un courant contenant des métaux lourds et les métaux sont précipités sous forme de sulfures métalliques.

**11.** Procédé selon la revendication 10, dans lequel les sulfures métalliques sont ensuite traités pour produire des métaux et du soufre élémentaire, et le soufre élémentaire est recyclé à l'étape de réduction dans le bioréacteur.

**12.** Procédé selon la revendication 10 ou 11, dans lequel le métal lourd comprend du cuivre et/ou du plomb.

# Fig 1

# Fig 2